Europäisches Patentamt

European Patent Office

Office européen des brevets

(19)

(11) Numéro de publication: **0 115 368**
**A2**

(12) **DEMANDE DE BREVET EUROPEEN**

(21) Numéro de dépôt: **84200090.3**

(22) Date de dépôt: **24.01.84**

(51) Int. Cl.³: **G 01 N 21/90**

(30) Priorité: **01.02.83 IT 8551683**

(43) Date de publication de la demande:
**08.08.84 Bulletin 84/32**

(84) Etats contractants désignés:
**AT BE CH DE FR GB IT LI LU NL SE**

(71) Demandeur: **Pilastro, Gelsomino**
**Via Preazzi, 26**
**I-36030 Costabissara(IT)**

(72) Inventeur: **Pilastro, Gelsomino**
**Via Preazzi, 26**
**I-36030 Costabissara(IT)**

(74) Mandataire: **Bettello, Luigi, Dott. Ing.**
**Via Col d'Echele, 25**
**I-36100 Vicenza(IT)**

(54) Machine automatique pour le contrôle électronique du contenu des fioles et flacons de produits pharmaceutiques.

(57) Les fioles (4) à contrôler sont amenées pas à pas au poste de vérification, après une rotation qui impartit un mouvement de vortex au liquide contenu. Une télécaméra (5) envoie les images de chaque fiole à un appareillage de contrôle (6) qui possède en mémoire l'image de la fiole échantillon, la comparaison réalisée provoquant l'evacuation automatique des fioles dont le liquide renferme des impuretés.

FIG. 3

EP 0 115 368 A2

Croydon Printing Company Ltd.

Machine automatique pour le contrôle électronique du contenu des fioles et flacons de produits pharmaceutiques

La présente invention a pour objet une machine à fonctionnement automatique propre à assurer le contrôle de l'intégrité, du niveau et de la pureté du liquide contenu dans les fioles, ampoules ou flacons en verre utilisés dans l'industrie pharmaceutique.

Une caractéristique principale de la machine suivant l'invention réside dans le fait que le contrôle automatique des fioles ou similaires à l'aide d'une télécaméra est effectué alors que les fioles individuelles, après une succession de rotations mécaniques, sont maintenues en avant de l'objectif de ladite télécaméra entre les mandrins et contre-mandrins d'un ensemble d'entraînement comportant une table mise en rotation à des intervalles de temps déterminés. Le contrôle du contenu des fioles est obtenu en comparant une ou plusieurs images de chacun des types de fioles mémorisés dans l'appareillage électronique et le signal qui provient de la télécaméra et qui correspond à l'image subséquente de la fiole contrôlée.

Suivant une autre caractéristique de la machine suivant l'invention, la source d'éclairage des fioles est constituée par un rayon laser dont le diamètre varie en fonction de celui de la fiole observée, étant noté que ce rayon peut être orienté soit de manière directement axiale en entrant par le fond de chaque fiole, soit obliquement de façon presque parallèle à l'axe de ladite fiole lorsque celle-ci est pourvue à ses extrémités de fonds opaques.

Sur le dessin annexé ;

Fig. 1 est une vue en perspective montrant l'ensemble d'une machine de contrôle suivant l'invention.

Fig. 2 est une vue de côté avec coupe partielle du dispositif placé à la sortie de la trémie d'alimentation pour opérer l'agitation des fioles à contrôler.

Fig. 3 représente le schéma de l'appareillage électronique.

Fig. 4 est une coupe verticale du mécanisme à mandrins et contre-mandrins pour l'entraînement en rotation des fioles.

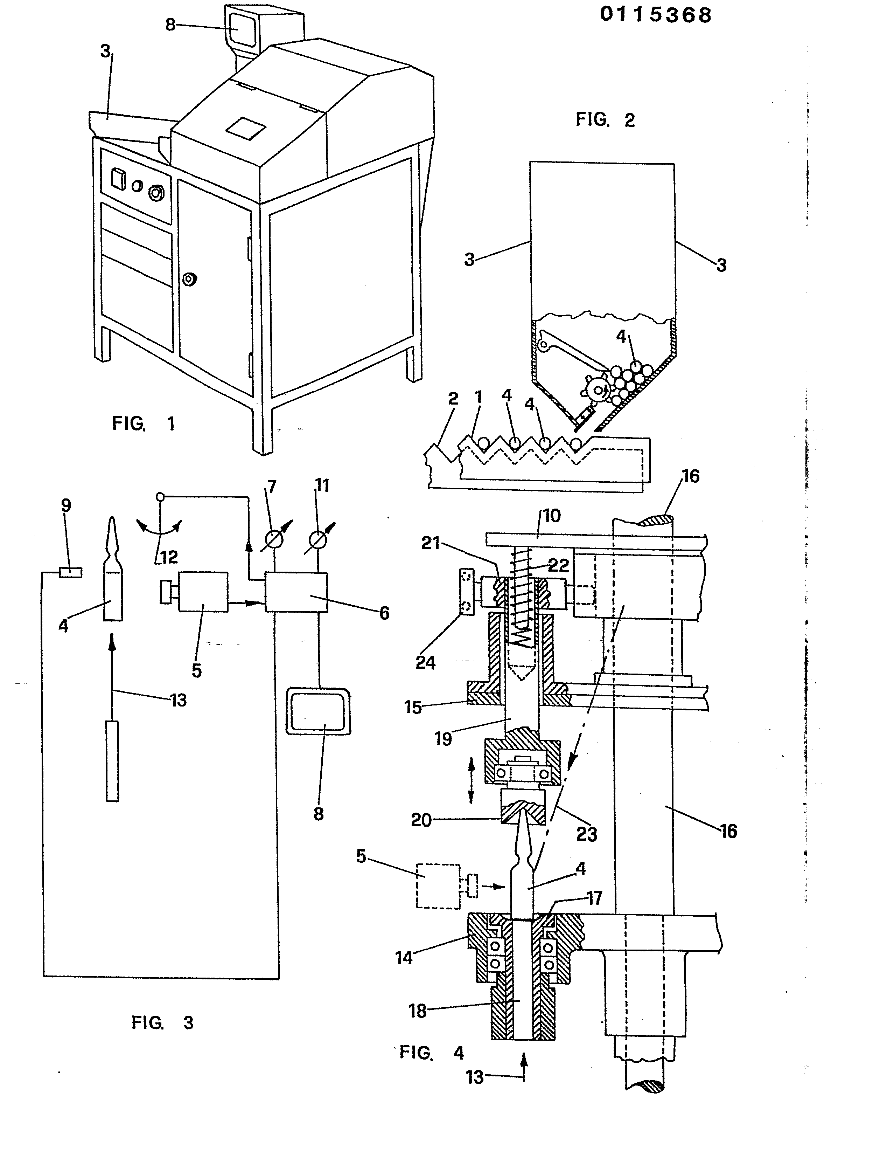

La machine représentée en fig. 1 comprend un dispositif d'alimentation en fioles, constitué par exemple par deux séries 1 et 2 de lames dentées fixes, respectivement mobiles (fig. 2) qui prélèvent une par une les fioles contenues dans une trémie 3 et qui les amènent pas à pas au poste où est effectué le contrôle du contenu de chaque fiole.

Dans les deux postes qui précèdent le poste de contrôle, chaque fiole est mise en rotation à la vitesse qui a été choisie par l'opérateur lors de la phase de mise au point en fonction des dimensions des fioles 4 et de la nature du liquide qui y est contenu. Les particules éventuellement renfermées par la fiole sont en conséquence entraînées, sous l'effet de la force centrifuge, vers l'extérieur de celle-ci et sont ainsi détachées du fond. Le liquide affecte de la sorte un mouvement de vortex, avec déplacement depuis le haut vers le bas au niveau de la périphérie de la fiole, et depuis le bas vers le haut au niveau du centre de celle-ci. Les gouttes éventuelles de liquide en suspension dans le goulot ou col, dans l'extrémité supérieure en pointe ou au bord de la fiole sont entraînées vers le bas, si bien que c'est tout le contenu qui est remélangé et mis en rotation.

Une fois que l'entraînement en rotation a cessé et alors que le liquide tourne encore sur lui-même, la fiole 4 passe au poste de lecture. C'est à ce poste qu'intervient l'éclairage à l'aide d'un rayon laser 13 (fig. 3 et 4) orienté axialement à travers le fond ou, dans le cas où l'on a affaire à des flacons à bouchons opaques, au moyen d'un tel rayon laser 23 (fig. 4) orienté obliquement de manière pratiquement parallèle à l'axe de ceux-ci.

L'appareillage de contrôle entre alors automatiquement en fonctionnement, suivant le cycle suivant : la télécaméra 5 envoie un signal vidéo à l'appareillage électronique 6 de fig. 3 qui le mémorise. Dans la mémoire de l'appareillage on envoie une ou plusieurs images lues par la télécaméra 5 afin d'obtenir une définition parfaite de l'immage réelle qui se présente en vis-à-vis de celle-ci.

Par la suite et pendant un temps qui peut être choisi, le signal vidéo qui provient de la télécaméra est comparé avec celui qui est en mémoire, et ce point par point.

On comprend que si des particules sont en mouvement dans la fiole contrôlée par effet d'inertie après la mise en rotation précédente, l'image qui se présente à la télécaméra est différente de l'image échantillon mémorisée. Grâce au régulateur de sensibilité 7 (fig. 3) il est possible de choisir le seuil d'intervention du circuit électronique qui compare l'image vidéo avec l'image mémorisée, de telle sorte que si en un point quelconque de la surface contrôlée il existe un signal de niveau supérieur au seuil choisi, une mémoire intervient qui retient un signal d'erreur.

Ceci signifie qu'une particule est présente à l'intérieur de la fiole observée et que celle particule comporte des dimensions supérieures à celles tolérées.

Sur l'écran de l'appareil moniteur 8 sont envoyées les mêmes images que celles qui atteignent l'appareillage de contrôle. Pour permettre à l'opérateur de contrôler le fonctionnement de la machine, on a prévu sur l'écran un réticule de points lumineux de référence qui sont normalement éclairés et qui s'éteignent lorsqu'un point correspondant de l'image dépasse le seuil intégré dans le régulateur 7. Ceci permet le contrôle de la sensibilité lors de la phase de mise au point et la surveillance de l'état opérationnel du système tout au long du fonctionnement normal.

Dans le système de mémoire est inséré un circuit particulier qui contrôle le nombre de points mémorisés. Lorsque ce nombre dépasse un chiffre prédéterminé, le circuit précité intervient et émet automatiquement un signal d'erreur, de la même manière que dans le cas où il existe des particules en mouvement.

En effet en pareil cas l'éclairage excessif est du à un effet du verre ou à la détérioration et cristallisation du liquide. La fiole est alors éliminée.

Lorsqu'une fiole 4 se trouve en position de lecture et au moment même où commence la comparaison des images, intervient également un détecteur photoélectrique 9 destiné au contrôle du niveau du liquide. Ce détecteur est constitué par un photo-transistor à positionnement et sensibilité réglables.

Le détecteur 9 est braqué sur la zone d'interruption du liquide, où par effet du rayon laser il se forme une bande fortement lumineuse. La lumière émise est reçue par le photo-transistor et en absence d'une telle émission la fiole correspondante est éliminée pour erreur de dosage, étant observé qu'un régulateur 11 peut, lorsque désiré, permettre de choisir la tolérance.

Lorsque les fioles contrôlées se trouvent à la position d'évacuation, un déviateur électro-mécanique 12 (fig. 3) intervient pour assurer, en fonction des données de l'appareillage 6, l'envoi des fioles vers les sorties "bon" ou "rebut".

Le nombre des fioles est compté de manière automatique, avec décompte des "bon", des "rebut" systématiques et des "rebut" pour erreur du niveau du liquide contenu, ceci pour le contrôle statistique de la production.

Comme mentionné ci-dessus, les fioles ou flacons sont éclairés par rayon laser soit axialement à travers le fond, soit transversalement suivant une direction pratiquement parallèle à l'axe dans le cas des flacons à bouchons opaques. La lumière est du type monochromatique et elle est émise suivant un faisceau très restreint qui, en fonction du diamètre des fioles à contrôler, peut être élargi afin de les éclairer complètement.

Si ce rayon rencontre une particule à l'intérieur du liquide contenu, on assiste de manière simultanée, successive ou alternée, à des phénomènes de diffusion, réflexion ou réfraction, en fonction de l'inclinaison de la particule par rapport au rayon incident. Les particules en suspension et en mouvement présentent au rayon laser, à l'entrée et à la sortie, des surfaces différentes qui provoquent ainsi les phénomènes précités.

La télécaméra 5, disposée à 90° par rapport à l'axe de la fiole, se trouve dans la position idéale pour recueillir la lumière réfractée, réfléchie ou diffusée par les particules éventuelles. Des corps qui ne sont pas naturellement réfléchissants, tels que des poils, des parties gommeuses ou autres, sont facilement détectés par suite de la réfraction très intense qui est obtenue.

Du fait que les phénomènes décrits ci-dessus produisent des radiations lumineuses pratiquement constantes, à parité d'angle d'incidence et de type de matériel, jusqu'à une dimension de l'ordre de dix fois la longueur d'onde du rayon d'origine, on peut en déduire qu'à l'aide d'un laser à émission de $0,65 \mu$m, il est possible de visualiser des particules de dimansions aussi minimes que $6,5 \mu$ m.

La puissance du laser utilisé est relativement réduite et elle ne présente des danger pour l'oeil humain que lorsque le rayon est observé de manière directe avec insistance.

En ce qui concerne au contraire l'effet sur une particule, les choses sont bien différentes : le rayon peut varier de 2 à 10 mm de diamètre avec une puissance de 5 mW. On obtient qu'au moyen d'un rayon de 2 mm une particule (supposée

sphéroidale) de 100 $\mu$ m de diamètre est investie par une portion de rayon de la puissance de 250$\mu$W, laquelle est bien supérieure à celle susceptible d'être obtenue à l'aide d'une lampe traditionnelle. Avec un rayon de 10 mm la même particule est investie par une portion de rayon éclairant de la puissance de 40 $\mu$W, qui est toujours considérablement supérieure à celle obtenue avec une telle lampe traditionnelle de puissance raisonnable.

Fig. 4 montre l'ensemble mécanique destiné à supporter et à faire tourner les fioles à observer. Cet ensemble comprend deux disques tournants 14 et 15 montés sur un arbre vertical 16, le disque inférieur 14 comportant, pour l'appui des fioles, plusieurs mandrins 17, par exemple huit, creusés d'un alésage axial 18 et portés à rotation par des roulements à billes. C'est à travers chaque alésage axial 18 que passe le rayon laser 13 sus-mentionné lorsqu'on doit illuminer les fioles axialement par le dessous.

Pour soutenir la pointe des fioles 4 on a monté à travers le disque supérieur 15, suivant des axes qui coïncident avec les huit mandrins 17, un nombre égal de tiges 19 dont l'extrémité inférieure porte un contre-mandrin 20. Celui-ci est monté à rotation sur des roulements à billes et il comporte un logement interne à profil conique propre à recevoir l'extrémité pointue d'une fiole, laquelle sera ainsi mise en mouvement avant l'observation électronique automatique.

Chaque tige 19 est sollicitée élastiquement dans le sens axial du fait qu'elle est reliée à un bras radial 21 par l'intermédiaire d'un ressort 22 prenant appui contre une entretoise circulaire supérieure 10, laquelle est rendue solidaire de l'arbre vertical 16 de la machine au-dessus du disque 15.

Dans ces conditions on conçoit qu'en surmontant le léger effort exercé par le ressort 22, à la manière indiquée par la flèche qui apparaît en fig. 4, il est possible de soulever une fiole 4 avant le contrôle, et pour l'en retirer après que celui-ci ait été effectué. Le soulèvement du contre-mandrin 20 est assuré par une came tournante qui élève périodiquement le roulement à billes 24 porté par chaque bras radial 21 lors de sa rotation sur l'arbre 16.

L'ensemble rotatif principal formé par les disques 14 et 15 et les mandrins et contre-mandrins qui leur sont associés est entraîné à des intervalles de temps égaux, de façon à permettre les temps d'arrêt nécessaires aux opérations de prélèvement, de double rotation des fioles, d'éclairage du contenu de celles-ci à l'aide du rayon laser et de contrôle, telles que ci-dessus décrites.

Il convient d'observer que la machine suivant l'invention est susceptible d'être immédiatement adaptée à tous les types de fioles, flacons et autres emballages transparents utilisés en pharmacie ou industrie similaire. Son originalité réside aussi bien dans l'utilisation d'un rayon laser comme source d'éclairage du contenu des fioles que dans le contrôle automatique à l'aide de l'appareillage électronique qui compare l'image de la fiole examinée relevée par la télécaméra avec celle du même type de fiole antérieurement mémorisée à titre d'échantillon, et ce de manière entièrement automatique, sans aucune intervention manuelle.

Revendications

1. Machine automatique pour le contrôle électronique du contenu des fioles ou flacons de produits pharmaceutiques, caractérisée en ce qu'elle comprend un ensemble mécanique qui tourne avec un arbre (16) orienté de manière substantiellement verticale, et qui est formé par deux disques parallèles (14, 15) équipés de mandrins (17) et de contre-mandrins (20) entre lesquels viennent automatiquement s'engager les extrémités des fioles (4) dont on désire vérifier l'intégrité du liquide contenu, ces fioles étant éclairées une par une par un rayon laser (13 ou 23).

2. Machine suivant la revendication 1, caractérisée en ce que le contrôle automatique des fioles ou autres flacons (4) est opéré à l'aide d'une télécaméra (5) qui envoie dans la mémoire d'un appareillage électronique (6) au moins une image de la fiole en cours de contrôle, le signal vidéo provenant de cette télécaméra braquée sur ladite fiole étant ensuite comparé avec celui en mémoire correspondant à un échantillon type.

3. Machine suivant l'une quelconque des revendications 1 et 2, caractérisée en ce que le rayon laser d'éclairage (13) pénètre dans chaque fiole (4) par le fond de celle-ci, à travers un alésage (18) ménagé axialement dans chaque mandrin (17).

4. Machine suivant l'une quelconque des revendications 1 et 2, caractérisée en ce que pour le contrôle automatique du contenu des fioles ou autres récipients (4) équipés de bouchons opaques à leurs extrémités, l'éclairage à l'aide d'un rayon laser (23) de diamètre opportun est effectué suivant une direction oblique, pratiquement parallèle à l'axe desdits récipients.

5. Machine suivant l'une quelconque des revendications 1 à 4, caractérisée en ce que le contrôle de l'intégrité du contenu des fioles (4) qui se trouvent au poste de lecture est opéré en même temps qu'une vérification du niveau du liquide, cette vérification étant effectuée à l'aide d'un détecteur photo-électrique (9) qui admet une tolérance de réglage du niveau.

0115368

FIG. 1

FIG. 2

FIG. 3

FIG. 4